# FASCICULE DE BREVET EUROPEEN

(11) **EP 0 714 266 B1**
(45) Date de publication et mention de la délivrance du brevet: **21.10.1998**
(21) Numéro de dépôt: 94922307.7
(22) Date de dépôt: 26.07.1994
(51) Int. Cl.: A61B 8/12, A61B 19/00, A61B 17/36, G10K 11/00, A61N 7/02

(54) **SONDE ENDOCAVITAIRE DE THERAPIE ET D'IMAGERIE ET APPAREIL DE TRAITEMENT THERAPEUTIQUE EN COMPORTANT APPLICATION**
ENDOSKOPISCHE SONDE ZUR ABBILDUNG UND THERAPIE UND IHR BEHANDLUNGSSYSTEM
IMAGING AND THERAPY INTRALUMINAL PROBE AND THERAPEUTIC TREATMENT APPARATUS UTILIZING SAME

(30) Priorité: 26.07.1993 FR 9309158; 04.02.1994 FR 9401304
(43) Date de publication de la demande: 05.06.1996
(73) Titulaire: TECHNOMED MEDICAL SYSTEMS, 69500 Bron (FR); INSTITUT NATIONAL DE LA SANTE ET DE LA RECHERCHE MEDICALE (INSERM), 75643 Paris Cédex 13 (FR)
(72) Inventeur: DORY, Jacques, F-77580 Villiers-sur-Morin (FR); CONSTANTINOU, Georges, F-94210 La Varenne-Saint-Hilaire (FR); CHAPELON, Jean-Yves, F-69100 Villeurbanne (FR); BLANC, Emmanuel, F-69230 Saint-Genis-Laval (FR); LACOSTE, François, F-69006 Lyon (FR); GILLES, Gérard, F-77440 Congis-sur-Therouanne (FR)
(74) Mandataire: Hirsch, Marc-Roger
(86) Numéro de dépôt international: FR9400936
(87) Numéro de publication internationale: WO9502994

(56) Documents cités:
- EP-A- 0 088 620
- EP-A- 0 273 180
- WO-A-89/07909
- WO-A-90/02520
- WO-A-92/15253
- DE-A- 3 813 298
- DE-A- 3 910 336
- DE-C- 4 119 524
- INTERNATIONAL JOURNAL OF CARDIAC IMAGING, vol.4, no.2-4, 1989, DORDRECHT (NL) pages 79 - 88 N.BOM ET AL. 'Early and recent intraluminal ultrasound devices'

## Description

La présente invention a pour objet une sonde endo-cavitaire de thérapie (ou traitement) et d'imagerie (ou visualisation), comportant un corps de sonde, au moins un transducteur d'imagerie pour l'émission d'ondes d'imagerie et au moins un transducteur de thérapie pour l'émission d'ondes de thérapie, montés mobiles dans la dite sonde, des moyens d'entraînement dudit transducteur d'imagerie et des moyens d'entraînement du dit transducteur de thérapie.

Elle a aussi pour objet un appareil de traitement thérapeutique en comportant application.

Dans l'état de la technique, il est connu depuis fort longtemps des appareils de traitement thérapeutique comprenant la combinaison d'un transducteur de thérapie et d'un transducteur d'imagerie (ou visualisation), habituellement de type extracorporel. On peut se reporter à ce sujet au document DORY US-A 4 658 828.

Les dispositifs de traitement extracorporels antérieurement connus donnent généralement satisfaction mais toutes les voies d'accès ne sont pas toujours possibles. D'autre part, il peut être difficile avec un dispositif de traitement extracorporel de réaliser un traitement de tissus situés en profondeur du corps d'un être humain et dans la mesure où les ondes ultrasoniques de thérapie traversent des zones tissulaires saines qu'il faut préserver. De ce fait, il est généralement souhaitable d'utiliser des sondes endocavitaires pour un traitement plus précis et sûr seulement des tissus à soumettre au traitement.

Il existe déjà des sondes de traitement ou thérapie, ainsi que des sondes de traitement et d'imagerie.

Le document EP-A-0 317 049 décrit une sonde ultrasonique, présentant une fenêtre entourant un transducteur ultrasonique. Le transducteur ultrasonique est monté sur une première plate-forme circulaire susceptible de pivoter autour d'un de ses diamètres. Cette plate-forme est montée sur une deuxième plate-forme circulaire susceptible de tourner dans son plan, autour de son axe. De la sorte, le transducteur ultrasonique est susceptible de balayer des secteurs angulaires dans tous les plans. La sonde du document EP-A-0 317 049 est destinée à l'imagerie des tissus environnant la sonde.

Le document FR-A-2 673 542 décrit une sonde endorectale de thérapie et un appareil de destructions de tissus tumoraux, en particulier de la prostate, en comportant application. L'appareil de destruction selon ce document comprend une telle sonde endorectale et une sonde endo-urétrale d'échographie. La sonde endorectale de thérapie comprend un transducteur piézo-électrique ayant une face avant d'émission d'ondes acoustiques ultrasoniques, monté dans un organe support lié à un moyen guide rigide permettant une introductions endorectale de la sonde. L'ensemble ainsi constitué est entouré d'une membrane déformable en silicone ou en latex, susceptible d'être rempli de liquide après introduction de la sonde.

Le document FR-A-2 673 542 propose une solution au problème de la destruction de tissus tumoraux permettant un suivi en temps réel. La solution proposée consiste à utiliser une sonde endo-urétrale d'imagerie par échographie.

La solution proposée présente des inconvénients. Elle implique tout d'abord l'utilisation simultanée d'une sonde endo-urétrale et d'une sonde endorectale, ce qui augmente le traumatisme. Il est aussi nécessaire de déplacer les deux sondes, pour les besoins de l'échographie et du traitement. Ceci engendre des traumatismes supplémentaires. La précision de l'appareil est limitée par la précision mécanique des moyens de support communs de la sonde endo-rectale et de la sonde endo-urétrale. Pour obtenir une bonne précision, il est nécessaire d'utiliser des systèmes mécaniques complexes. La sonde endorectale en cas de rupture de son enveloppe en latex ou en silicone, peut provoquer des traumatismes importants du fait de ses mouvements. Enfin la combinaison d'une sonde endorectale et d'une sonde endo-urétrale ne permet pas une visualisation en temps réel, le transducteur d'échographie étant saturé en réception lors de l'émission de l'onde de traitement.

Le document WO-A-89/07909 décrit, en particulier en référence à sa figure 5, une sonde endorectale de localisation et de thérapie. Cette sonde comprend un transducteur focalisant susceptible d'émettre des ultrasons pour l'imagerie ou la visualisation. Ce transducteur est monté sur une tige mobile par rapport à l'enveloppe extérieure souple de la sonde. La sonde comprend en outre un transducteur focalisant pour la thérapie, qui est monté pivotant autour d'un de ses diamètres sur des bras mobiles par rapport à l'enveloppe extérieure souple de la sonde. Le transducteur de thérapie émet en direction d'un miroir de renvoi qui réfléchit les ultrasons vers la zone a traiter.

Ce dispositif présente des inconvénients. Il est d'un volume important, du fait du nombre des éléments. Il comprend en outre des dispositifs mécaniques nombreux, pour lesquels il est difficile d'obtenir une précision importante. Le document présente en outre le même danger que la sonde du document EP-A-0 317 049, en cas de déchirement de l'enveloppe souple externe.

La combinaison dans la sonde endocavitaire de ce document d'un transducteur de thérapie et d'un transducteur d'imagerie implique des solutions complexes, notamment dans la mesure où le transducteur de thérapie émet des ondes ultrasoniques qui doivent être réfléchies sur un miroir de renvoi sur les tissus à traiter. En raison de la présence de ce miroir, il est impossible de trouver un rapport d'ouverture optimal pour une concentration optimale des ondes ultrasoniques à la zone focale étant entendu que l'ouverture du transducteur de thérapie est donnée par le rapport diamètre/distance focale. De ce fait, dans le cadre de ce dispositif antérieur, il est physiquement impossible d'obtenir une ouverture du transducteur de thérapie voisine de 1 ou même supérieure à 1. Il en résulte une limitation supplémentaire des performances thérapeutiques.

La présente invention pallie ces inconvénients. Elle fournit une solution simple, sûre, peu traumatisante pour l'imagerie et le traitement des tissus; elle est particulièrement adaptée au traitement de la prostate.

La présente invention, notamment par rapport à l'art antérieur constitué par le document WO-A-89/07909, permet de limiter l'encombrement de la sonde, sans diminuer l'efficacité et la précision du traitement et de l'imagerie.

La présente invention a aussi pour but de résoudre le nouveau problème technique consistant en la fourniture d'une solution permettant de fournir une sonde endocavitaire combinant un transducteur de thérapie et un transducteur d'imagerie, dans laquelle le transducteur de thérapie présente une ouverture définie par le rapport diamètre/distance focale, le plus grand possible, si possible voisin de 1 ou même supérieur à 1, en obtenant ainsi une concentration maximale des ondes ultrasoniques à la zone focale, avec une efficacité grondement accrue de la thérapie.

Dans un mode de réalisation, la présente invention a encore pour but de résoudre le nouveau problème technique consistant en la fourniture d'une solution permettant de fournir une sonde endocavitaire combinant un transducteur de thérapie et un transducteur d'imagerie, permettant de réduire le périmètre de la section de la sonde endocavitaire dans un plan perpendiculaire à l'axe d'introduction, tout en permettant de maintenir une ouverture optimale du transducteur de thérapie, ainsi que simultanément, de positionner le transducteur d'imagerie en lieu et place du transducteur de thérapie pour obtenir une image des tissus à traiter dans une position unique du corps de sonde endocavitaire, que ce soit pour l'imagerie ou pour la thérapie.

Dans un mode de réalisation, la présente invention a en outre pour but de résoudre le nouveau problème technique consistant en la fourniture d'une solution permettant de fournir une sonde endocavitaire combinée en transducteur de thérapie en transducteur d'imagerie, à ouverture optimale pour un traitement efficace, dans laquelle le transducteur de thérapie est limité en mouvement et notamment ne réalise qu'un mouvement de rotation en particulier autour d'un axe de rotation parallèle à l'axe d'introduction de la sonde endocavitaire, pour une meilleure précision de la thérapie. Avantageusement, ce problème technique doit être résolu selon une solution qui évite une translation du corps de la sonde endocavitaire entre la phase d'imagerie et la phase de thérapie pour éviter tout risque de déplacement de la zone tissulaire à traiter, telle qu'un organe.

La présente invention a encore pour but de résoudre les nouveaux problèmes techniques énoncés précédemment selon une solution qui permet d'utiliser une sonde d'imagerie indépendante, en permettant ainsi l'emploi de tout type de sonde d'imagerie commerciale, en réduisant ainsi pratiquement le coût de fabrication.

La présente invention permet pour la première fois de résoudre simultanément les problèmes techniques énoncés ci-dessus, d'une manière simple, fiable, reproductible, relativement peu coûteuse, utilisable à l'échelle industrielle et médicale.

L'invention propose une sonde endocavitaire de thérapie et d'imagerie, selon les revendications annexées.

Grâce à l'invention, la sonde présente une structure mécanique simple, un encombrement réduit, et peut comprendre un transducteur de thérapie à grande ouverture.

En outre, il est facile de passer du mode de traitement au mode d'imagerie, et réciproquement. Ceci n'implique aucun mouvement de la sonde, et garantit une excellente précision de traitement.

La dite sonde endocavitaire présente une forme allongée définissant un axe général d'introduction dans la cavité du corps et est caractérisée en ce que :
- le transducteur d'imagerie est monté sur des premiers moyens supports assurant une translation sensiblement parallèlement audit axe général de la sonde endocavitaire du transducteur d'imagerie entre une position rétractée et une position avancée;
- le transducteur de thérapie est monté sur des seconds moyens supports assurant une rotation du transducteur de thérapie selon un axe de rotation sensiblement parallèle à l'axe général de la sonde endocavitaire mais décalé par rapport au transducteur d'imagerie, de préférence hors de l'empattement du transducteur d'imagerie, pour permettre une rotation du transducteur de thérapie entre une position escamotée dite de repos pour laquelle le transducteur d'imagerie peut être déplacé en translation dans ladite position avancée et venir occuper la place précédemment occupée par le transducteur de thérapie et une position de traitement pour laquelle le transducteur d'imagerie est en position de retrait, afin de permettre la formation d'une image avec le transducteur d'imagerie de la zone cible destinée à être traitée par le transducteur de thérapie, en permettant ainsi de visualiser et de traiter la zone cible à partir de la même position de la sonde endocavitaire.

Selon un mode de réalisation avantageux, le transducteur de thérapie et le transducteur d'imagerie sont disposés à l'intérieur d'une membrane souple remplie d'un liquide de couplage acoustique tel que l'eau ou une huile.

Selon un autre mode de réalisation avantageux, le transducteur de thérapie est monté dans un organe support solidaire des seconds moyens supports, présentant une section transversale dans un plan perpendiculaire à l'axe d'introduction définissant un périmètre qui est aussi proche que possible du périmètre de la section d'un même plan du transducteur de thérapie.

Selon encore un autre mode de réalisation avantageux, le transducteur de thérapie présente une forme extérieure en disque à profil en majeure partie sensiblement circulaire mais ayant un diamètre réduit dans le sens perpendiculaire à l'axe d'introduction.

Selon encore un autre mode de réalisation avantageux, le transducteur d'imagerie est prévu pour fournir des images dans plusieurs plans, par exemple deux plans perpendiculaires, un premier plan dit radial perpendiculaire à l'axe d'introduction et un second plan dit sagittal contenant sensiblement ou parallèlement l'axe d'introduction en particulier permettant aussi de réaliser l'image de la zone à traiter pendant le traitement.

Selon un autre mode de réalisation avantageux, le transducteur de thérapie est prévu pour réaliser une focalisation selon une direction sensiblement perpendiculaire à l'axe général d'introduction dans la cavité du corps.

Selon un mode de réalisation particulièrement avantageux, l'organe support précité du transducteur de thérapie présente une forme extérieure sensiblement circulaire ou polygonale, voisine de la forme extérieure du transducteur de thérapie qui est elle-même sensiblement circulaire ou polygonale, l'organe support du transducteur de thérapie étant solidaire des seconds moyens supports précités au voisinage d'un bord, de manière à faciliter le déplacement du transducteur de thérapie entre la position escamotée précitée et la position de traitement.

Selon un mode de réalisation actuellement préféré, le transducteur de thérapie présente une forme extérieure définissant un axe de symétrie qui est sensiblement parallèle à l'axe d'introduction précité.

Selon une variante de réalisation avantageuse, lorsque le transducteur de thérapie présente une forme extérieure en disque à profil en majeure partie sensiblement circulaire, et en particulier dans la variante éventuelle où il présente un diamètre réduit dans le sens perpendiculaire à l'axe d'introduction, ledit transducteur présentant un axe de symétrie qui est sensiblement parallèle à l'axe d'introduction précité.

Selon une variante de réalisation avantageuse de l'invention, le transducteur d'imagerie fait partie d'une sonde d'imagerie indépendante qui de préférence est constituée par tout type de sonde commerciale qui avantageusement permet de fournir des images dans plusieurs plans, en particulier deux plans, comme précédemment décrit.

Selon une autre variante de réalisation avantageuse de l'invention, le transducteur de thérapie présente une ouverture, c'est-à-dire un rapport diamètre/ distance focale compris entre 0,8 et 1,5 et encore mieux d'environ 1, en fournissant ainsi l'avantage déterminant pour la thérapie d'une efficacité accrue à la zone focale et l'absence de lésion secondaire sur les tissus situés entre le transducteur de thérapie et la zone focale.

Selon une autre variante de réalisation avantageuse de l'invention, des moyens de mise en rotation manuelle ou motorisée du transducteur de thérapie ainsi que des moyens de mise en translation manuelle ou motorisée du transducteur d'imagerie sont prévus, éventuellement cette rotation ou cette translation étant réalisée par l'intermédiaire des premier et second moyens supports précités.

Le transducteur de traitement peut aussi comprendre une lentille déformable mécaniquement permettant de modifier sa focale.

On assure ainsi une plage de traitement plus importante.

Dans un mode de réalisation, la sonde de traitement et d'imagerie comporte une paroi rigide entourant au moins partiellement lesdits transducteurs.

Ceci évite tout danger pour le patient du fait du mouvement des transducteurs.

Dans ce cas, ladite paroi rigide peut comporter une fenêtre.

Cette fenêtre permet un passage des ultrasons vers le patient.

Dans un mode de réalisation de l'invention, la sonde de traitement et d'imagerie comporte une enveloppe souple localement déformable.

Cette enveloppe assure un contact avec la zone à traiter et/ou à visualiser, et permet ainsi le passage des ultrasons.

L'invention couvre également un appareil de traitement, caractérisé en ce qu'il comprend une sonde endocavitaire telle que précédemment définie.

Selon un mode de réalisation particulier de cet appareil de traitement, celui-ci est un appareil de traitement d'une zone cible à l'intérieur du corps d'un mammifère, en particulier un être humain, comprenant des moyens d'introduction de la sonde endocavitaire dans une cavité du corps. Cette cavité peut être naturelle ou être provisoirement créée par voie chirurgicale, notamment par incision.

Dans un mode de réalisation particulier, la zone cible est une zone de tissus à traiter, en particulier des tissus malins et bénins, habituellement sous forme de tumeurs dites bénignes ou malignes.

Selon un autre mode de réalisation particulier de l'invention, l'appareil de traitement précité est un appareil de traitement des tissus de la prostate par voie urétrale ou rectale. Dans le cadre du traitement de cancer de la prostate, la voie rectale est actuellement préférée.

L'invention peut encore s'appliquer pour un traitement d'une zone cible à l'intérieur du corps d'un mammifère, en particulier d'un être humain, avec les étapes suivantes:
- la prévision d'une sonde endocavitaire présentant une forme allongée définissant un axe général d'intro-duction dans une cavité du corps ;
- la prévision d'un transducteur d'imagerie monté sur des premiers moyens de supports assurant une translation parallèlement audit axe général de la sonde endocavitaire du transducteur d'imagerie entre une position rétractée et une position avancée ;
- la prévision d'un transducteur de thérapie monté sur des seconds moyens supports assurant une rotation du transducteur de thérapie selon un axe de rotation parallèle à l'axe général de la sonde endocavitaire mais décalé par rapport au transducteur d'imagerie, de préférence hors de l'empattement du transducteur d'imagerie, pour permettre une rotation du transducteur de thérapie entre une position escamotée dite de repos pour laquelle le transducteur d'imagerie peut être déplacé en translation dans ladite position avancée et venir occuper la place précédemment occupée par le transducteur de thérapie et une position de traitement pour laquelle le transducteur d'imagerie est en position de retrait,
- l'introduction de la sonde endocavitaire dans ladite cavité du corps jusqu'à ce que le transducteur de thérapie soit disposé sensiblement en regard de ladite zone cible,
- le cas échéant, la mise en rotation du transducteur de thérapie pour le positionner dans ladite position escamotée de repos,
- le déplacement en translation du transducteur d'imagerie dans la position avancée pour venir occuper la place du transducteur de thérapie,
- la réalisation de la formation d'une image avec le transducteur d'imagerie de la zone cible à traiter par le transducteur de thérapie, permettant de visualiser la zone cible,
- le retrait du transducteur d'imagerie dans ladite position de retrait,
- la mise en rotation du transducteur de thérapie pour l'amener dans la position de traitement,
- l'émission d'ondes ultrasoniques de traitement pendant une période de temps prédéterminée qui est nécessaire pour réaliser ce traitement.

Les caractéristiques et avantages de l'invention apparaîtront à la lecture de la description suivante d'un mode de réalisation de la sonde selon l'invention, donné à titre d'exemple et en référence aux figures annexées, qui montrent:
- la figure 1 une vue schématique d'ensemble d'un premier mode de réalisation d'une sonde selon l'invention et de son mécanisme de support;
- la figure 2 une vue à plus grande échelle de la tête de la sonde de la figure 1;
- la figure 3 une vue plus détaillée en coupe d'un transducteur de traitement muni d'un réglage de focalisation.
- la figure 4 est une vue schématique d'un appareil de traitement selon la présente invention comportant une sonde endocavitaire selon l'invention avec ses organes de commande essentiels ;
- la figure 5 représente une vue en coupe axiale longitudinale de la sonde endocavitaire selon un mode de réalisation de la présente invention présentant la partie avant et la partie arrière de ladite sonde endocavitaire, en position d'introduction ou de traitement ;
- la figure 6 est une vue en coupe similaire à celle de la figure 5 mais comportant un arrachement partiel au niveau des moyens de commande en rotation du transducteur de thérapie, après une rotation de 90° par rapport à la position de la figure 5 du transducteur de thérapie et positionnement du transducteur d'imagerie en position avancée;
- la figure 7 représente schématiquement les deux types de plan de coupe du transducteur d'imagerie de la sonde selon les figures 4 à 6;
- la figure 8 représente schématiquement le début de l'introduction de la sonde endocavitaire selon les figures 4 à 7 dans une cavité du corps d'un être humain, ici le rectum ;
- la figure 9 représente une vue schématique en coupe représentant la position imagerie du transducteur d'imagerie relativement a la prostate d'un patient à traiter, ledit patient étant en position sur le côté, après rotation de 90° par rapport à la position de la figure 4 où le patient est sur le dos ; et
- la figure 10 représente une vue similaire à la figure 9, mais en position de traitement pour laquelle la sonde de traitement a son point focal disposé dans le volume à traiter T.V.

L'invention a pour objet une sonde endocavitaire de thérapie et d'imagerie, comportant un corps de sonde, au moins un transducteur d'imagerie pour l'émission d'ondes d'imagerie et au moins un transducteur de thérapie pour l'émission d'ondes de thérapie, montés mobiles dans la dite sonde, des moyens d'entraînement du dit transducteur d'imagerie et des moyens d'entraînement du dit transducteur de thérapie. Cette sonde est caractérisée en ce que le dit transducteur de thérapie et le dit transducteur d'imagerie sont montés mobiles dans la sonde de telle sorte que le dit transducteur de thérapie lors de l'émission des ondes de thérapie émet directement les ondes de thérapie suivant une direction sensiblement identique à la direction suivant laquelle le dit transducteur d'imagerie émet les ondes d'imagerie lors de l'émission des ondes d'imagerie.

Du fait que le transducteur de thérapie émet directement les ondes de thérapie suivant une direction de tir identique ou sensiblement identique à la direction des tirs d'imagerie, il n'est pas nécessaire de disposer d'un miroir de renvoi. La structure mécanique de la sonde est simplifiée. De plus, du fait que la sonde comprend moins de pièces en mouvement relatif, la précision du traitement et la corrélation entre le traitement et la visualisation sont augmentées. L'invention permet notamment d'augmenter l'efficacité du traitement, et de limiter l'encombrement de la sonde.

Dans les modes de réalisation de l'invention, décrits en référence aux figures 4 à 8, les moyens d'entraînement des transducteurs de thérapie et de visualisation sont indépendants.

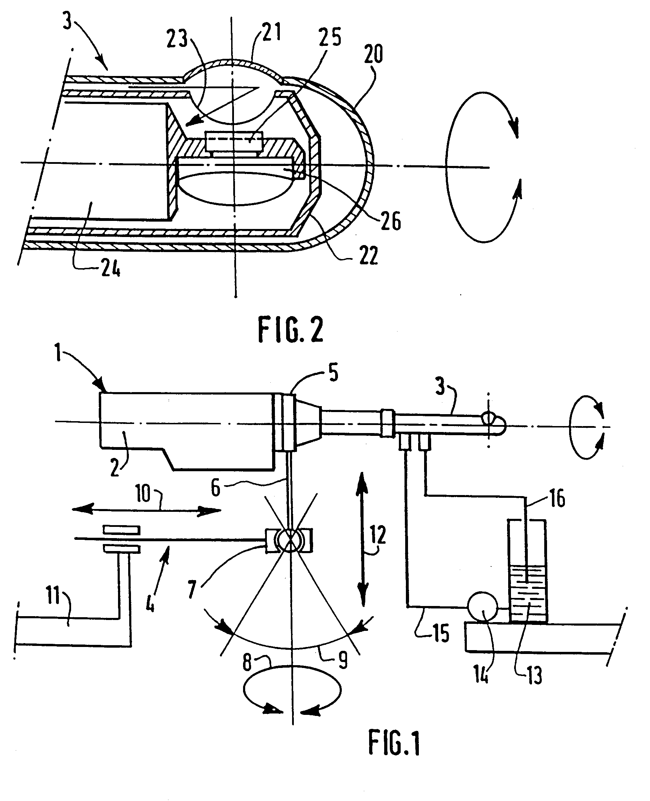

La figure 1 montre une vue schématique d'ensemble d'une sonde selon l'invention et de son mécanisme de support. la sonde 1 présente un corps 2 et une tête 3 destinée à être introduite dans le patient. Le corps de la sonde est relié à un mécanisme de support 4, qui permet de déplacer la sonde 1 et/ou de la maintenir en une position donnée. Le mécanisme de support peut présenter une forme quelconque. Dans le mode de réalisation de la figure 1 le mécanisme de support comprend un collier 5 dans lequel le corps 2 de la sonde, est monté rotatif, et qui est relié à une extrémité d'une tige rigide 6. L'autre extrémité de la tige 6 est mobile dans un joint à rotule 7 ce qui permet de déplacer la sonde en rotation autour de la tige 6, ou en pivotant autour du joint rotule, comme indiqué par les règles 8 et 9 de la figure 1. Le joint rotule 7 est monté libre en translation suivant la flèche 10 de la figure 1, sur un support 11. Le support 11 est susceptible de se déplacer verticalement dans le plan de la figure, comme indiqué par la flèche 12.

On a représenté à la figure 1 un réservoir 13 de liquide, associé à une pompe 14. Le liquide refoulé par la pompe 14 est amené par un conduit 15 à la sonde 1. Le circuit du liquide dans la sonde l sera expliqué plus en détail en référence à la figure 2. Le liquide provenant de la sonde 1 retourne au réservoir 13 par un conduit 16.

La figure 2 montre une vue à plus grande échelle de la tête de la sonde de la figure 1. La sonde présente une enveloppe souple 20 présentant une zone déformable 21. Cette enveloppe souple est susceptible d'être démontée et par exemple changée pour chaque patient. La sonde présente une paroi 22 rigide qui présente une fenêtre 23. La paroi 22 présente une forme allongée et est recouverte par l'enveloppe souple 20. La zone déformable 21 de l'enveloppe souple est disposée en regard de la fenêtre 23 de la paroi rigide. Dans le mode de réalisation décrit en référence aux figures 1 et 2, la fenêtre 23 est disposée sur le côté de la sonde. D'autre modes de réalisation sont possibles, comme cela apparaît clairement des figures suivantes. La fenêtre 23 pourrait faire le tour de la sonde, ou s'étendre partiellement sur le pourtour de celle-ci.

A l'intérieur de la paroi rigide 22 la sonde comprend un un transducteur d'imagerie et un transducteur de traitement. Le mode de réalisation particulier de ces transducteurs sur la figure 2 (transducteurs dos à dos et axe de rotation commun) n'est pas selon l'invention.

Dans la présente description, les transducteurs permettent d'émettre des ultrasons, afin de visualiser ou de traiter le patient. Les techniques d'imagerie et/ou de traitement par ultrasons sont décrites dans l'art antérieur et sont connues de l'homme du métier.

Le transducteur d'imagerie est par exemple, constitué d'un transducteur piézo-composite d'un diamètre de 10 mm, de rayon focal de 45 mm, avec une fréquence de résonance de 7,5 MHz. Le transducteur de traitement présente une forme concave et focalise les ultrasons. Il peut par exemple être constitué d'un transducteur de 25 mm de diamètre, d'un rayon focal de 50 mm, avec une fréquence de résonance de 3,5 MHz.

La circulation du liquide dans la sonde 1, mentionnée plus haut, s'effectue de la façon suivante. Le liquide en provenance de la pompe est refoulé entre la paroi rigide 22 et l'enveloppe élastique 20. Il circule à travers la fenêtre 23, pénètre à l'intérieur de la paroi rigide 22 et s'écoule vers le réservoir en passant entre l'arbre 24 et la paroi 23. L'écoulement de fluide dans la sonde permet d'assurer un refroidissement des transducteurs. Il permet aussi d'assurer un gonflage de la zone déformable 21, de façon à ce que celle-ci entre en contact avec les tissus du patient. Le contact assure le passage vers la zone à traiter des ultrasons émis par les transducteurs.

La sonde des figures 1 et 2 est particulièrement adapté au traitement de la prostate, par voie rectale. A titre d'exemple d'utilisation on décrit ci-dessous le mode opératoire pour le traitement de la prostate. La sonde fonctionne de la façon suivante: la sonde est d'abord introduite dans le rectum du patient, de façon à ce que la fenêtre 23 soit au voisinage de la prostate. L'enveloppe souple est alors gonflée par envoi de liquide par la pompe. La zone déformable de la membrane 21 vient en contact avec les tissus du patient, permettant ainsi le passage des ultrasons vers la zone à traiter et/ou à visualiser. On procède ensuite à la visualisation de la zone à traiter. Pour cela, on utilise le transducteur d'imagerie. On peut utiliser le transducteur d'imagerie en échographie mode A, en plaçant le transducteur face à la fenêtre 23 par rotation de l'arbre 24, et en le maintenant immobile. On peut aussi assurer un balayage échographique par rotation de l'arbre 24. Ceci permet d'obtenir des images en échographie B. La largeur du balayage dépend de la taille de la fenêtre 23, qui peut être adaptée. Dans le mode de réalisation de la figure 1, les images sont prises dans un plan orthogonal à l'axe de la sonde.

Une fois les zones à traiter visualisées et localisées, on passe en mode de traitement. Ceci n'implique aucun mouvement de la sonde. L'invention permet ainsi de pallier le manque de précision des dispositifs de l'art antérieur: la précision d'un déplacement mécanique de la sonde.

L'invention fournit aussi un dispositif d'une grande sûreté. Même si l'enveloppe souple 20 se déchire ou se rompt, les pièces en mouvement éventuel, à savoir l'arbre 24 supportant les transducteurs, restent à l'intérieur de la paroi rigide; elles ne risquent donc pas de blesser le patient.

Selon l'invention, on peut à volonté et dans un temps très court, passer du mode de traitement au mode d'imagerie. Le traitement peut consister en des émissions d'ultrasons de traitement, alternés avec des émissions d'ultrasons d'imagerie, ce qui permet de suivre en temps réel la progression du traitement. On peut aussi choisir de ne visualiser la zone traitée que de temps à autre, en guise de vérification.

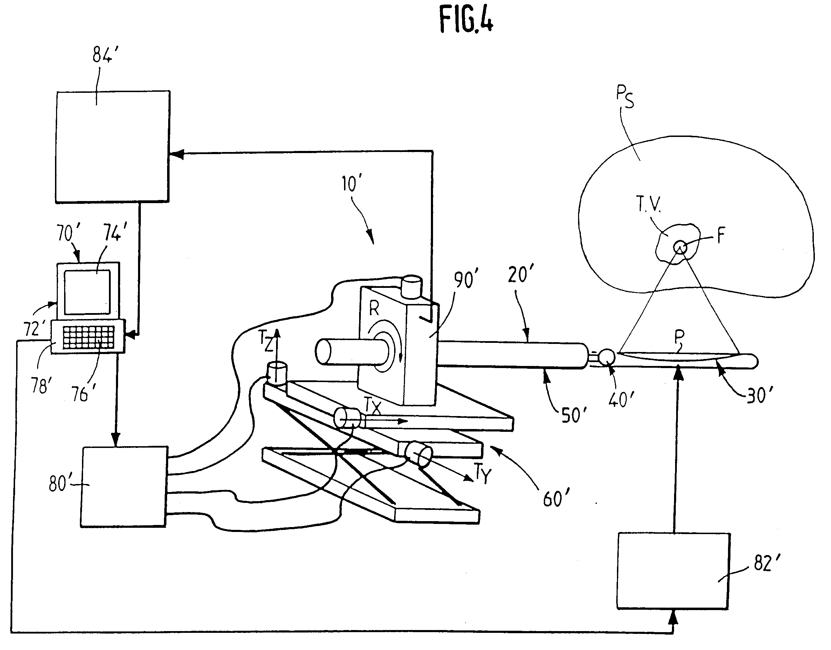

Selon un mode de réalisation de l'invention, le transducteur de thérapie est muni sur sa face d'émission d'une poche de liquide d'indice différent et qui est susceptible d'être plus ou moins gonflée et de faire ainsi varier la distance focale du transducteur. Cette poche agit en combinaison avec la déformation de la zone déformable 21 de l'enveloppe souple 20 pour faire varier la distance focale du transducteur de thérapie. On peut ainsi grâce à l'invention soigner des tumeurs à différentes profondeurs sans déplacer mécaniquement la sonde. Ceci apparaîtra plus clairement encore de la description de la figure 3.

La figure 3 montre une vue plus détaillée d'un transducteur de thérapie muni d'une lentille de réglage de la focalisation selon l'invention. Le transducteur 60 comprend une surface émettrice 61, constituée par exemple d'une pastille piézo-électrique. Une membrane élastique 62 transparente aux ultrasons est tendue devant la surface émettrice 61. Un liquide 63 se trouve entre la surface émettrice 61 et la membrane élastique 62.

Le liquide 63 présente une vitesse de propagation différente de celle du milieu extérieur dans lequel les ultrasons vont se propager. Dans le cas d'une sonde telle que celle décrite en référence aux figures 1 ou 2, le milieu extérieur est constitué par de l'eau, le liquide 63 présente un indice ou une vitesse de propagation différent de celui ou celle de l'eau. Des moyens de pression, tels qu'une pompe, non représentés, peuvent faire varier la quantité de liquide 63 de façon à modifier la forme de la membrane 62. Ceci conduit à une modification de la distance focale du transducteur 60. Sous l'action de la pression du liquide 63, la membrane 62 prend une forme de calotte quasi sphérique.

La pastille 61 présente en son centre un évidement 64, et un dispositif piézo-électrique de petite dimension 65 est disposé en regard de l'évidement. Le dispositif piézo-électrique 65 permet de connaître la position de la membrane 62 au droit de l'évidement 64, par calcul du temps de parcours dans le liquide 63 d'une impulsion ultrasonore. A l'aide de cette position on peut, connaissant le diamètre de la surface émettrice 61, déterminer le rayon de la calotte sphérique formée par la membrane 62, et calculer la distance focale du transducteur 60. On pourrait aussi déterminer la distance focale à partir de la mesure de la pression du liquide 63 de gonflage de la membrane, sur la base d'un étalonnage préalable. Bien entendu, le transducteur 60 comprend des moyens d'excitation de la surface émettrice 61 et du dispositif piézo-électrique 65, non représentés.

Il est possible de donner à la surface émettrice 61 une forme quelconque. Elle peut par exemple être concave, ce qui limite pour une focale donnée la déformation de la membrane 62.

Le transducteur 60 est susceptible d'être utilisé dans une sonde selon l'invention, telle que celles des figures 1 ou 2.

Le transducteur de la figure 3 permet de faire varier la focale d'un transducteur, et d'effectuer des traitements à des profondeurs différentes, et ce sans déplacer le transducteur ni modifier la taille de la surface d'entrée des rayonnements ultrasonores dans la zone à traiter. Ceci présente l'avantage d'éviter les appareillages mécaniques et limite le risque de brûlure.

A titre d'exemple, on peut utiliser une pastille 61 d'un diamètre de l'ordre de 35 mm, présentant un évidement de 4 mm de diamètre en son centre. La présence du réglage de focalisation permet de traiter à des profondeurs variant de 5 à 40 mm, soit des focales de 15 à 50 mm.

On décrit maintenant divers processus de thérapie possibles à l'aide de sondes selon l'invention. Dans un premier mode de thérapie, on effectue un tir à l'aide de transducteur de thérapie, immobile. On entraîne ensuite en rotation le transducteur d'imagerie pour effectuer un balayage et une visualisation de la zone traitée. On arrête ensuite la rotation des transducteurs pour procéder à un nouveau tir de thérapie, et ainsi de suite.

Dans un second mode de thérapie, on procède à intervalles réguliers à des balayages par le transducteur d'imagerie, de façon à obtenir des images de la zone à traiter. Entre ces balayages, on procède à une pluralité de tirs de thérapie. Pendant chaque tir de thérapie, le transducteur de thérapie est immobile. Entre chaque tir de thérapie, on peut éventuellement déplacer le transducteur de thérapie ou faire varier sa focale, de façon à détruire une zone étendue.

Dans tous les modes de réalisation de l'invention, le transducteur de thérapie peut être plan ou présenter une préfocalisation du fait de sa forme. Il peut être muni d'un dispositif de focalisation mécanique, tel que celui décrit à la figure 3. Il peut aussi être muni, le cas échéant, d'un dispositif de focalisation électronique.

De même, l'invention n'est pas limitée aux formes et tailles de fenêtres décrites ci-dessus. La paroi rigide pourrait être transparente aux ultrasons, et la fenêtre ne serait alors pas nécessaire.

En référence aux figures 4 à 8, on a représenté un appareil de traitement selon la présente invention selon un mode de réalisation actuellement préféré, représenté par le numéro de référence général 10'. Cet appareil de traitement comprend, de manière générale, un dispositif formant sonde endocavitaire 20' comprenant au moins un transducteur de thérapie 30' et au moins un transducteur d'imagerie 40' montés sur des moyens supports tels que 50', 60'. Cet appareil de traitement comprend, généralement, une centrale de commande 70', en général comprenant un ordinateur ou micro-ordinateur 72' avec tous les dispositifs périphériques classiques, tels qu'un écran vidéo 74', un clavier d'introduction de données 76', une mémoire active et/ou passive 78'. Cette centrale de commande 70' commande des moyens de commande 80' en translation et/ou en rotation des divers moyens et/ou dispositifs de l'appareil de traitement, et notamment les moyens 82' de commande d'émission d'ondes ultrasoniques de thérapie au transducteur de thérapie 30', comme cela est bien compréhensible pour un homme de l'art. En outre, on peut prévoir que les données d'imagerie fournies par le transducteur d'imagerie 40' sont transmises à un moyen d'affichage d'image 84' tel qu'un écran vidéo, qui sont ensuite transférées à la centrale de commande 70'.

Dans le cadre de l'invention, il est avantageux que la sonde endocavitaire 20' puisse être montée en rotation autour de son axe longitudinal par des moyens de mise en rotation 90' commandés par les moyens de commande 80' comme symbolisé par la flèche R. Ces moyens de mise en rotation 90' sont avantageusement montés sur une table de déplacement dans la direction X, Y et Z comme cela est symbolisé par les flèches TX, TY et TZ. De telles tables sont bien connues à l'homme de l'art, ainsi que les moyens de commande correspondants 80' et ne sont donc pas décrits plus en détail ici.

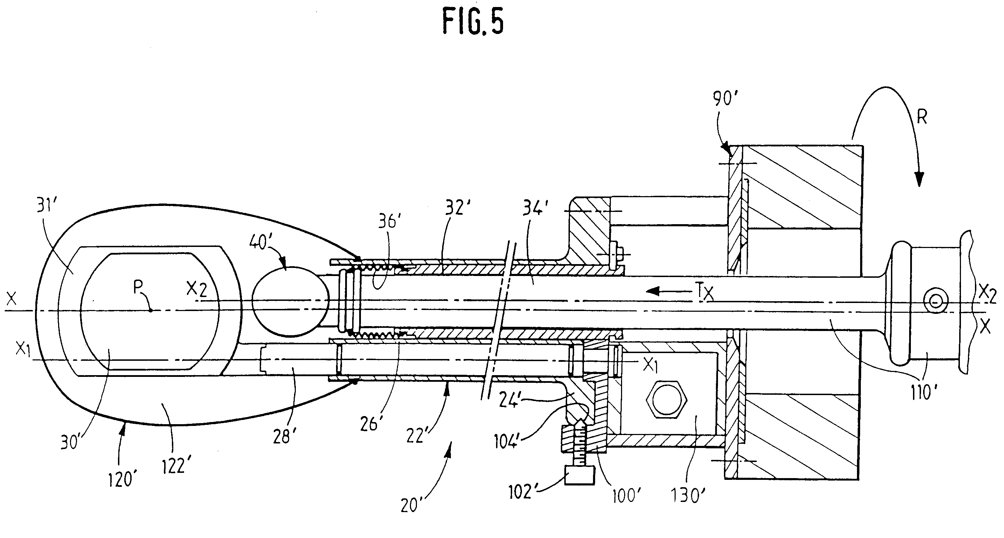

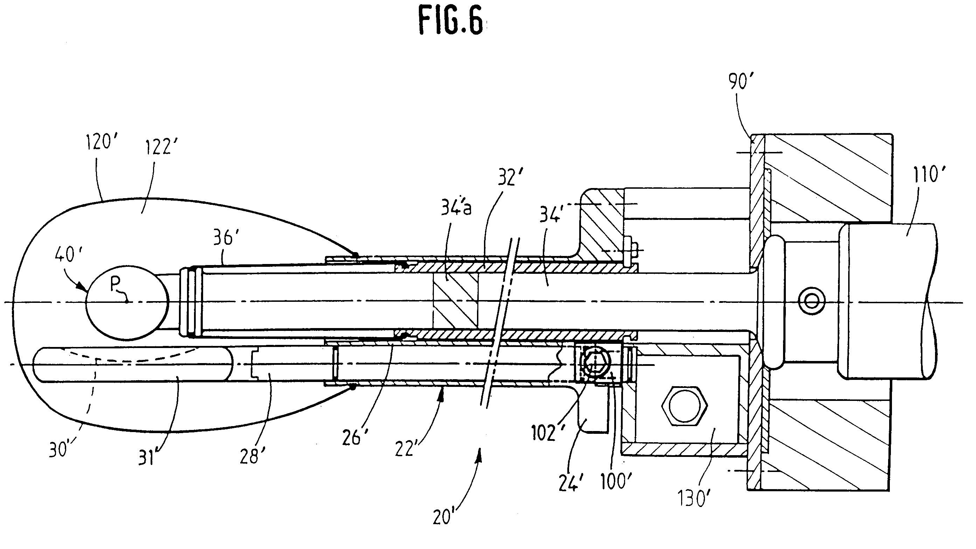

La sonde endocavitaire est représentée plus en détail en coupe longitudinale aux figures 5 et 6.

Cette sonde endocavitaire 20' comprend généralement un corps 22' de forme allongée sensiblement tubulaire comportant à sa base arrière un flasque 24' de connexion au moyen de mise en rotation 90' de la figure 4 pour permettre une commande en rotation de la sonde 20' autour de son axe longitudinal X-X.

Le corps 22' de la sonde endocavitaire 20' comprend à l'intérieur un premier élément tubulaire 26', d'axe X1-X1 décalé par rapport à l'axe longitudinal X-X de la sonde endocavitaire 20' et servant de logement et de guidage en rotation à une tige 28' formant moyen support de mise en rotation du transducteur de thérapie 30'.

D'autre part, ce corps 22' comprend un deuxième élément tubulaire 32' comprenant un axe X2-X2 qui peut être coaxial ou légèrement décalé de l'axe X-X de la sonde endocavitaire 20', comme représenté. Ce deuxième tube 32' sert de logement et de guidage en translation d'une tige 34' support du transducteur d'imagerie 40'.

Par ailleurs, le transducteur de thérapie 30' est monté en rotation autour de l'axe X1-X1 par l'intermédiaire de sa tige support 28' par des moyens de mise en rotation individuelle 100' pouvant être bloqués en position par des moyens de blocage 102' tels qu'une vis coopérant avec un logement correspondant 104' dans le flasque 24', une première position dite de traitement utilisée aussi pour l'introduction telle que représentée à la figure 5 et une deuxième position dite escamotée telle que représentée à la figure 6' après rotation d'un angle de rotation de 90° autour de l'axe X1-X1 pour relever le transducteur de thérapie 30' dans une position sensiblement perpendiculaire au plan défini par la position de traitement de la figure 5. On observera ainsi qu'ici les moyens de mise en rotation 100' peuvent être commandés manuellement. Ils peuvent naturellement être commandés de manière automatique par la centrale de commande 70' selon une autre variante de réalisation grâce à des moyens de commande en rotation motorisés bien connus à l'homme de l'art.

La tige 34' support du transducteur d'imagerie 40' peut être commandée en translation individuelle comprenant la tige 110' qui peut être commandé manuellement ou également par des moyens de commande motorisés, qui ne sont pas représentés plus en détail ici car leur réalisation est bien connue à l'homme de l'art.

Lorsque l'on veut garantir la réalisation uniquement d'une translation du transducteur d'imagerie 40' entre la position rétractée, représentée à la figure 5 et la position avancée, représentée à la figure 6, on peut prévoir que le second élément tubulaire 32' présente en coupe une section non circulaire, par exemple une section rectangulaire ou carrée, et dans ce cas la tige 34' support du transducteur d'imagerie 40' présentera une section complémentaire, par exemple une section rectangulaire telle que représentée schématiquement en 34'a à la figure 6. On peut prévoir avantageusement un élément 36' formant un joint d'étanchéité entre la tige 34' et le second élément tubulaire 32', relativement à un liquide 122' de couplage acoustique décrit ci-après, réaliser avantageusement sous la forme d'un soufflet pour s'adapter aux diverses positions de translation du transducteur d'imagerie, et pouvant éventuellement aussi exercer un effet ressort pour faciliter le retrait du transducteur d'imagerie dans la position de retrait de la figure 5. On peut également prévoir un boîtier 130' permettant de loger les éléments d'adaptation d'impédance électrique de l'élément transducteur de thérapie 30', qui peut être réalisé sous forme d'un circuit électronique, comme cela est bien connu a l'homme de l'art. Un tel dispositif d'adaptation d'impédance électrique peut aussi être solidaire de la sonde endocavitaire.

On observera par ailleurs que selon l'invention, le transducteur d'imagerie 40' peut être monté dans une sonde d'imagerie indépendante 34', comme représenté qui peut être constituée par tout type de sonde commerciale. De telles sondes d'imagerie commercialement disponibles sont bien connues à l'homme de l'art. Comme il a été dit précédemment, on préfère selon l'invention une sonde d'imagerie capable de former des images dans plusieurs plans, en particulier deux plans perpendiculaires.

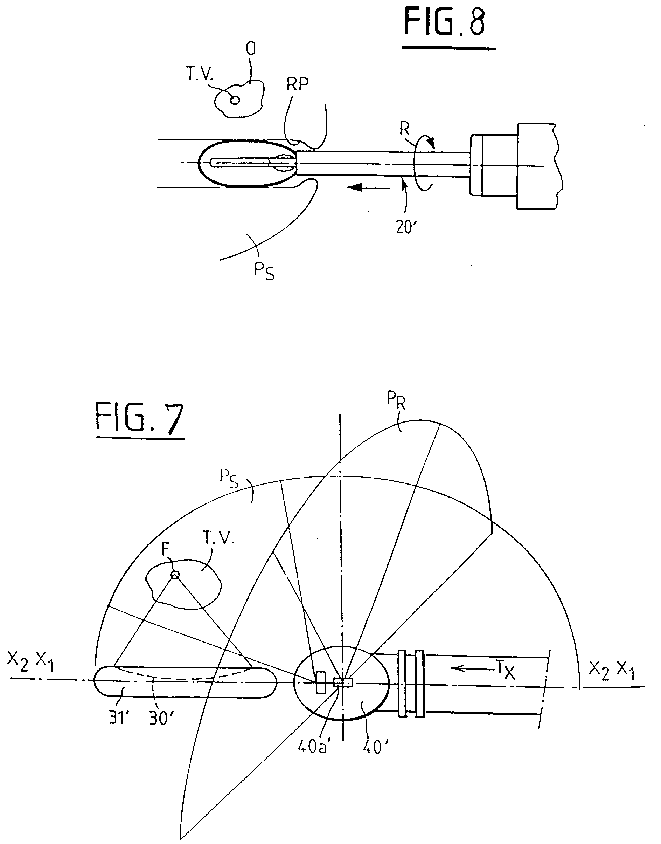

Ainsi, on observera à partir de la figure 5 que lorsque le transducteur d'imagerie 40' est dans sa position de retrait, il laisse la place au transducteur de thérapie qui peut être mis dans la position de traitement représentée à la figure 5. Après rotation de 90° à partir de la position d'imagerie de la zone cible représentée à la figure 6, pour permettre de faire avec le transducteur d'imagerie 40' en position de retrait, de l'imagerie de la zone cible en temps réel grâce à sa capacité de former des images dans deux plans ici perpendiculaires, ici dans le plan PS de la figure 7 comme cela sera décrit plus loin.

On comprend également que le transducteur de thérapie 30' dans sa position de traitement de la figure 5 présente un point central P qui est voisin du point central de positionnement de la sonde d'imagerie 40' dans sa position avancée de la figure 6, ce qui permet d'obtenir une image de la zone ou volume cible (T.V.) (TARGET VOLUME) dans une position voisine de la position du transducteur de thérapie 30' au moment de la thérapie.

On peut également prévoir que la sonde endocavitaire 20' se termine à sa partie avant par une membrane 120' souple définissant un volume intérieur dans lequel sont disposés déplaçables respectivement le transducteur de thérapie 30' et le transducteur d'imagerie 40', cette cavité étant remplie d'un liquide 122' de couplage acoustique tel que de l'eau, en particulier dégazée, ou une huile. La structure et le montage de cette membrane 120' sont similaires à ceux qui sont décrits dans le document antérieur des déposants FR-A-2 673 542 = PCT/EP-9200410 auquel l'homme de l'art pourra se reporter.

Également, le transducteur de thérapie 30' est de préférence constitué par celui décrit dans ce document antérieur FR-A-2 673 542 = PCT/EP-9200410, avec son organe support correspondant 31', pour obtenir les avantages de minimisation de volume dans une section transversale dans un plan perpendiculaire à l'axe d'introduction. Ainsi, le transducteur de thérapie est avantageusement monté dans l'organe support 31', solidaire des moyens supports, ici sous forme de tige 28', présentant une section transversale dans un plan perpendiculaire à l'axe d'introduction X-X définissant un périmètre qui est aussi proche que possible du périmètre de la section d'un même plan du transducteur de thérapie 30', comme cela a été décrit en détail dans le document précité antérieur.

Avantageusement aussi, le transducteur de thérapie 30' présente une forme extérieure en disque à profil en majeure partie sensiblement circulaire, mais ayant un diamètre réduit dans le sens perpendiculaire à l'axe d'introduction. On pourra, à ce sujet, se reporter aux figures 5 à 9 du document précité antérieur.

La sonde d'imagerie peut être toute sonde d'imagerie disponible dans le commerce.

Cependant, selon un mode de réalisation avantageux, le transducteur d'imagerie 40' est prévu pour fournir des images dites biplans, comme représenté à la figure 7, un premier plan dit radial PR perpendiculaire à l'axe d'introduction X-X grâce à un premier élément transducteur 40'a, et un second plan dit sagittal PS contenant l'axe ou sensiblement parallèle à l'axe d'introduction X-X en permettant ainsi de réaliser en particulier l'image de la zone à traiter T.V. pendant le traitement, comme cela est clairement compréhensible à partir de la considération des figures 4 et 7. Comme cela est également bien connu à l'homme de l'art, l'élément transducteur d'imagerie 40' est aussi monté en déplacement en rotation sur lui-même pour fournir le plan d'image PR ou PS.

On comprendra ainsi que l'invention permet de résoudre le problème technique précédemment énoncé et d'aboutir aux avantages techniques déterminants précédemment énoncés.

Il va être maintenant décrit le fonctionnement de l'appareil de traitement selon la présente invention avec mise en oeuvre du procédé de traitement précédemment décrit.

Cet appareil de traitement permet de réaliser le traitement de toute zone cible, en particulier d'une zone de tissus se trouvant à l'intérieur du corps d'un être vivant, en particulier un mammifère, avantageusement un être humain. Cette zone cible peut être une zone tissulaire, en particulier dans un organe, ces tissus pouvant être des tissus bénins ou malins, comme une tumeur bénigne ou maligne. L'invention trouve une application particulièrement intéressante dans le cas du traitement de tumeurs de la prostate. Dans ce cadre, on a représenté schématiquement à la figure 8 la phase d'introduction de la sonde endocavitaire 20' dans le rectum Rp d'un patient P par un mouvement de translation dans le sens de la flèche représentée à la figure 8. On peut également effectuer un mouvement de rotation R de la sonde endocavitaire 20' pour aboutir à un positionnement précis par rapport à un organe O tel que la prostate à l'intérieur de laquelle se trouve le volume tissulaire T.V. à traiter, telle qu'une tumeur bénigne ou maligne de cet organe O, par exemple la prostate. Dans cette phase d'introduction, le transducteur de thérapie 30' est positionné dans la position de la figure 5, la position d'introduction correspondant ainsi également à la position de traitement.

Lorsque ce positionnement précis est obtenu, le transducteur de thérapie 30' est positionné en position escamotée représentée à la figure 6' par l'actionnement des moyens de mise en rotation 100', puis la sonde d'imagerie 40' est avancée dans la position avancée représentée à la figure 6 pour réaliser une ou plusieurs images de la zone cible T.V., cette image étant affichée par les moyens d'affichage 84' et/ou 74' . Le volume de la zone cible T.V. peut être déterminé de manière automatique par la centrale de commande 70' grâce à des moyens de calcul intégrés, puis cette centrale de commande 70' peut commander de manière automatique, à partir de données éventuellement introduites par l'opérateur à l'aide du clavier 76' et d'instructions mémorisées dans une mémoire 78', la commande des moyens de commande 82' du transducteur de thérapie 30', après que celui-ci a été repositionné dans la position représentée à la figure 5 après retrait du transducteur d'imagerie 40', comme représenté à la figure 5.

Le traitement proprement dit de la zone cible T.V. par le transducteur de thérapie est bien connu à l'homme de l'art et n'a pas à être détaillé ici. Dans ce cadre, on pourra se reporter au document précédent FR-A-2 673 542 correspondant à PCT/EP-9200410. En particulier, l'élément transducteur de thérapie 30' peut être localisé naturellement ou par voie électronique et présenter un rapport de diamètre à la distance focale compris entre 0,8 et 1,5.

On peut faire émettre avantageusement à l'élément transducteur 30' des ondes ultrasoniques puisées à une fréquence comprise entre 1 et 4 MHz et avec une durée d'impulsion courte comprise entre 50 ms et 5 s et de préférence entre 1 et 4 s, l'intensité au point focal F, figure 4, étant comprise entre 1000 et 10000 W/cm².

Ce transducteur de thérapie 30' peut également être un transducteur piézo-électrique composite comme décrit dans le document précédent PCT/EP-9201534=FR-910997.

Pour réaliser le traitement complet de la zone cible T.V., par exemple une tumeur située dans l'organe O, il peut être nécessaire de déplacer la sonde endocavitaire 20' par un mouvement de rotation selon l'axe de rotation R et/ou par un mouvement de translation ici selon l'axe de translation TX comme cela est clairement visible et compréhensible à partir de la considération des figures.

D'autre part, les figures 4 à 8 font partie intégrante de la présente invention et donc de la présente description.

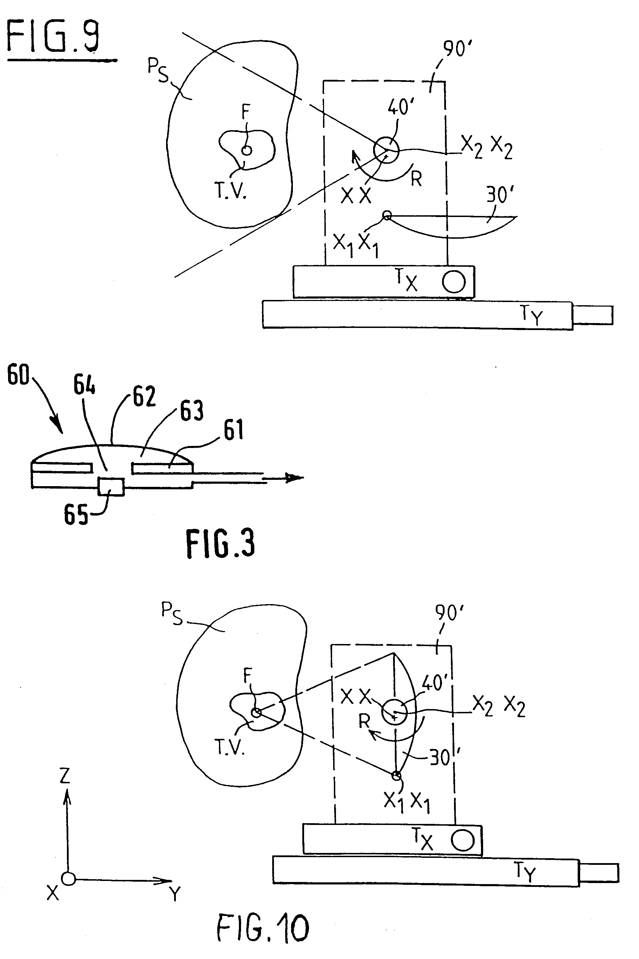

En référence à la figure 9, on a représenté la position d'imagerie de la sonde d'imagerie 40' dans sa position avancée, le transducteur de thérapie 30' étant disposé dans sa position escamotée après avoir effectué une rotation de 90° par rapport à la position d'introduction de la figure 5, position escamotée également représentée à la figure 6 pour l'imagerie préalable au traitement. Dans la position représentée à la figure 9, le patient est disposé sur le côté, après avoir été initialement disposé sur le dos comme représenté à la figure 4 au moment de l'introduction de la sonde endocavitaire, ici dans le rectum.

En référence à la figure 10, on a représenté la position de traitement qui est obtenue après avoir effectué un retrait par translation de la sonde d'imagerie 40' dans la position de retrait représentée à la figure 5, et une rotation de 90° du transducteur de thérapie 30' selon l'axe de rotation X1-X1, pour aboutir à la position représentée à la figure 5. Pour réaliser le traitement du volume de la tumeur T.V., on comprend que la zone focale F du transducteur de thérapie 30' localisant est trop petite pour effectuer ce traitement en une seule étape. Il est donc nécessaire de déplacer la zone focale en divers points pour obtenir le traitement total du volume de la tumeur. Ce déplacement est obtenu par mise en rotation de la sonde endocavitaire par une rotation selon la flèche R de la partie arrière 90' de la sonde endocavitaire 20', ce qui réalise un déplacement de la zone focale F selon l'axe Z tandis qu'un mouvement en translation selon l'axe TX et selon l'axe TY réalise un déplacement selon les axes X et Y, en permettant ainsi de traiter le volume T.V. dans l'espace X, Y, Z. Au cours de ce traitement, on peut observer que le transducteur d'imagerie 40' grâce à l'obtention du plan d'imagerie PS, permet de visualiser le volume à traiter T.V. ainsi que le point focal, en permettant de visualiser en temps réel le traitement ainsi réalisé, ce qui est déterminant pour une sécurité absolue du traitement.

Les modes de réalisation des figures 1 et 2 d'une part et 4 à 10 d'autre part peuvent avantageusement être combinés. On peut ainsi utiliser un transducteur de thérapie en forme de cuillère, telle que celui de la figure 5 ou 6, dans la sonde de la figure 1 ou 2. Les caractéristiques d'émission du transducteur de thérapie, décrites après la figure 10, peuvent s'appliquer au transducteur de traitement de l'invention, dans tous ses modes de réalisation. On peut aussi utiliser un transducteur de thérapie composite, présentant une focale variable électroniquement.

Bien entendu, les moyens de support de la sonde, ou le corps de sonde qui sont différents dans les divers modes de réalisation de l'invention, peuvent être échangés.

## Revendications

1. Sonde de thérapie et d'imagerie (10'), comportant
un corps de sonde (20') d'une forme allongée définissant un axe général (X-X),
au moins un transducteur d'imagerie (40') pour l'émission d'ondes d'imagerie, monté mobile dans la dite sonde (20'),
au moins un transducteur de thérapie (30') pour l'émission d'ondes de thérapie monté mobile dans la dite sonde,
le transducteur d'imagerie (40') permettant la formation d'une image de la zone cible (T.V.) destinée à être traitée par le transducteur de thérapie (30'),
caractérisée en ce que le transducteur d'imagerie (40') est monté mobile dans la dite sonde (20') sur des premiers moyens supports (26') assurant une translation sensiblement parallèlement audit axe général de la sonde du transducteur d'imagerie entre une position rétractée et une position avancée;
en ce que le transducteur de thérapie (30') est monté mobile dans la dite sonde sur des seconds moyens supports (28') assurant une rotation du transducteur de thérapie autour d'un axe de rotation (X1-X1) sensiblement parallèle à l'axe général (X-X) de la sonde mais décalé par rapport au transducteur d'imagerie;
en ce que lesdits seconds moyens supports assurent une rotation du transducteur de thérapie (30') entre une position de traitement pour laquelle le transducteur d'imagerie (40') est en position de retrait, et une position escamotée dite de repos pour laquelle le transducteur d'imagerie (40') peut être déplacé en translation dans ladite position avancée et venir occuper la place occupée par le transducteur de thérapie (30') dans sa position de traitement; et
en ce que le transducteur de thérapie (30') dans sa position de traitement peut émettre des ondes de thérapie directement suivant une direction sensiblement identique à la direction d'émission des ondes d'imagerie par le transducteur d'imagerie dans sa position avancée.

2. Sonde de thérapie et d'imagerie (10') selon la revendication 1, caractérisée en ce qu'elle présente des moyens d'entraînement (110') du dit transducteur d'imagerie (40') et des moyens d'entraînement (100') du dit transducteur de thérapie (30').

3. Sonde de thérapie et d'imagerie (10') selon la revendication 1 ou 2, caractérisée en ce que lesdits seconds moyens supports assurent une rotation du transducteur de thérapie (30') hors de l'empattement du transducteur d'imagerie (40').

4. Sonde de thérapie et d'imagerie selon l'une des revendications 1 à 3. caractérisée en ce que le transducteur de thérapie (30') et le transducteur d'imagerie (40') sont disposés à l'intérieur d'une membrane souple (120') remplie d'un liquide de couplage acoustique tel que de l'eau ou une huile.

5. Sonde de thérapie et d'imagerie selon l'une des revendications 1 à 4, caractérisée en ce que le transducteur de thérapie (30') est monté dans un organe support (31') solidaire des seconds moyens supports (28'), présentant une section transversale dans un plan perpendiculaire à l'axe général (X-X) de la sonde définissant un périmètre qui est aussi proche que possible du périmètre de la section dans le même plan du transducteur de thérapie (30').

6. Sonde de thérapie et d'imagerie selon la revendication 5, caractérisée en ce que le transducteur de thérapie (30') présente une forme extérieure en disque à profil en majeure partie sensiblement circulaire mais ayant un diamètre réduit dans le sens perpendiculaire à l'axe général de la sonde.

7. Sonde de thérapie et d'imagerie selon l'une des revendications 1 à 6, caractérisée en ce que le transducteur d'imagerie (40') est prévu pour fournir des images dans plusieurs plans (PR, PS), en particulier dans deux plans perpendiculaires, un premier plan (PR) dit radial perpendiculaire à l'axe général de la sonde et un second plan (PS) dit sagittal contenant ledit axe général, en particulier permettant aussi de réaliser une imagerie de la zone à traiter pendant le traitement.

8. Sonde de thérapie et d'imagerie selon l'une des revendications 1 à 7, caractérisée en ce que le transducteur de thérapie (30') est prévu pour réaliser une focalisation selon une direction sensiblement perpendiculaire au dit axe général (X-X).

9. Sonde de thérapie et d'imagerie selon l'une des revendications 1 à 8, caractérisée en ce que le transducteur de thérapie (30') est monté dans un organe support (31') présentant une forme extérieure sensiblement circulaire ou polygonale, voisine de la forme extérieure du transducteur de thérapie qui est elle-même sensiblement circulaire ou polygonale,
l'organe support du transducteur de thérapie étant solidaire des seconds moyens supports (28') au voisinage d'un bord, de manière à faciliter le déplacement du transducteur de thérapie (30') entre la position escamotée et la position de traitement.

10. Sonde de thérapie et d'imagerie selon la revendication 5, caractérisée en ce que le transducteur de thérapie présente une forme extérieure définissant un plan général contenant le dit axe général, dans la position de traitement.

11. Sonde de thérapie et d'imagerie selon l'une des revendications 1 à 10, caractérisée en ce qu'elle comprend des moyens de mise en rotation manuelle (100') ou motorisée du transducteur de thérapie (30') autour de son axe, ainsi que des moyens de mise en translation manuelle (110') ou motorisée du transducteur d'imagerie (40').

12. Sonde de thérapie et d'imagerie selon l'une des revendications 1 à 11, caractérisée en ce que le transducteur de thérapie présente un rapport de diamètre à la distance focale compris entre 0,8 et 1,5, et encore mieux environ 1.

13. Sonde de thérapie et d'imagerie selon l'une des revendications 1 à 12, caractérisée en ce que le transducteur de thérapie (30') émet des ondes ultrasoniques pulsées à une fréquence comprise entre 1 et 4 MHz et avec une durée d'impulsions courte comprise entre 50 ms et 5 s et de préférence entre 1 et 4s, l'intensité au point focal F étant comprise entre 1 000 et 10 000 W/cm².

14. Sonde de thérapie et d'imagerie selon l'une des revendications 1 à 13, caractérisée en ce que ledit transducteur de thérapie (60) comprend une lentille déformable mécaniquement (62) permettant de modifier sa focale.

15. Sonde de thérapie et d'imagerie selon l'une des revendications 1 à 14, caractérisée en ce qu'elle comporte une paroi rigide entourant au moins partiellement lesdits transducteurs.

16. Sonde de thérapie et d'imagerie selon la revendication 15, caractérisée en ce que ladite paroi rigide comporte une fenêtre.

17. Sonde de thérapie et d'imagerie selon l'une des revendications 1 à 16, caractérisée en ce qu'elle comporte une enveloppe souple localement déformable.

18. Appareil de traitement, comprenant une sonde de thérapie et d'imagerie telle que définie à l'une quelconque des revendications précédentes.

19. Appareil de traitement selon la revendication 18, pour le traitement d'une zone cible, en particulier une zone de tissus à traiter, et notamment des tissus malins ou bénins, habituellement sous forme de tumeurs dites bénignes ou malignes, à l'intérieur du corps d'un mammifère, en particulier d'un être humain, comprenant des moyens d'introduction de la dite sonde dans une cavité du corps.

20. Appareil de traitement selon l'une des revendications 18 ou 19, caractérisé en ce qu'il constitue un appareil de traitement des tissus de la prostate par voie urétrale ou rectale.

## Claims

1. A therapy and imaging probe (10') comprising:
a probe body (20') of elongated shape defining a general axis (X-X),
at least one imaging transducer (40') for transmitting imaging waves movably mounted in said probe (20'),
at least one therapy transducer (30') for transmitting therapy waves, mounted movably within said probe,
the imaging transducer (40') allowing an image of a target zone (T.V.) intended to be treated by said therapy transducer (30') to be formed,
characterized in that the imaging transducer (40') is movably mounted on said probe (20') on first support means (26') providing translational movement of the imaging transducer substantially parallel to the general axis of the imaging transducer probe, between a withdrawn position and an extended position;
in that the therapy transducer (30') is movably mounted in said probe on second support means (28') providing rotation of the therapy transducer about an axis of rotation (X1-X1) substantially parallel to the general axis (X-X) of the probe but offset with respect to the imaging transducer,
in that said second support means allow rotation of the therapy transducer (30') between a treatment position for which said imaging transducer (40') is in a withdrawn position, and a withdrawn or non-active position for which the imaging transducer (40') may be brought by translational movement to the extended position to occupy the place previously occupied by the therapy transducer (30') in its treatment position; and
in that said therapy transducer (30') in its treatment position can directly transmit said therapy waves in a direction substantially identical to the direction in which said imaging transducer transmits said imaging waves when in its advanced position.

2. A therapy and imaging probe according to claim 1, characterized in that it has driving means (110') for said imaging transducer (40') and driving means (100') for said therapy transducer (30').

3. A therapy and imaging probe according to claim 1 or 2, characterized in that said second support means provide rotation of said therapy transducer (30') outside the largest dimension of the imaging transducer (40').

4. A therapy and imaging probe according to one of claims 1 to 3, characterized in that the therapy transducer (30') and the imaging transducer (40') are arranged inside a flexible membrane (120') filled with an acoustic-coupling liquid such as water or an oil.

5. A therapy and imaging probe according to one of claims 1 to 4, characterized in that the therapy transducer (30') is mounted in a support member (31') integral with second support or driving means (28') having a cross-section in a plane perpendicular to the general axis (X-X) of the probe defining a perimeter which is as close as possible to the perimeter of a cross-section taken in the same plane of the therapy transducer (30').

6. A therapy and imaging probe according to claim 5, characterized in that the external shape of the therapy transducer (30') is that of a disc, the profile of which is for the major part substantially circular, but having a reduced diameter in a direction perpendicular to the general axis of the probe.

7. A therapy and imaging probe according to one of claims 1 to 6, characterized in that the imaging transducer (40') is arranged to provide images in several planes PR, PS), in particular in two perpendicular planes, comprising a first plane, or radial plane (PR), perpendicular to the general axis of the probe, and a second plane (PS), or sagittal plane, containing the general axis thereby making it, in particular, possible to also provide imaging of the treatment region during the course of treatment.

8. A therapy and imaging probe according to one of claims 1 to 7, characterized in that the therapy transducer (30') is arranged to provide focusing in a direction substantially perpendicular to the said general axis (X-X).

9. A therapy and imaging probe according to one of claims 1 to 8, characterized in that the therapy transducer (30') is mounted in a support member (31') having a substantially circular or polygonal outside shape, close to the outer shape of a substantially circular or polygonal-shaped therapy transducer, the therapy transducer support member being integral with the second support or driving means (28') close to an edge thereby facilitating movement of the therapy transducer (30') between the withdrawn position and a treatment position.

10. A therapy and imaging probe according to claim 5, characterized in that the outer shape of the therapy transducer defines a general plane containing the abovesaid axis of introduction, in the treatment position.

11. A therapy and imaging probe according to one of claims 1 to 10, characterized in that said probe comprises means (100') for providing manual rotation or motor-driven rotation of said therapy transducer (30'), and means (110') for providing manual or motor-driven translational movement of said imaging transducer (40').

12. A therapy and imaging probe according to one of claims 1 to 11, characterized in that the therapy transducer has an aperture ratio, in other words a ratio between diameter and focal length comprised between 0.8 and 1.5, and more preferably around 1.

13. A therapy and imaging probe according to one of claims 1 to 12, characterized in that said therapy transducer (30') delivers ultrasound waves pulsed at a frequency comprised between 1 and 4 MHz and with a short pulse duration comprised between 50 ms and 5 s and preferably between 1 and 4 s, with an intensity at a focal spot F comprised between 1,000 and 10,000 W/cm².

14. A therapy and imaging probe according to one of claims 1 to 13, characterized in that said therapy transducer (60) comprises a mechanically-deformable lens (62) allowing modification of the focal length thereof.

15. A therapy and imaging probe according to one of claims 1 to 14, comprising a rigid wall at least partially surrounding said transducers.

16. A therapy and imaging probe according to claim 15, characterized in that said rigid wall includes a window.

17. A therapy and imaging probe according to one of claims 1 to 16, comprising a locally-deformable flexible casing.

18. Treatment apparatus, comprising a therapy and imaging probe as defined in any one of the preceding claims.

19. Treatment apparatus according to claim 18, for the treatment of a target region, particularly a tissue region to be treated, in particular malignant or non-malignant tissue, usually in the form of non-malignant or malignant tumors, inside the body of a mammal, in particular a human being, comprising means for introducing a said probe into a body cavity.

20. Treatment apparatus according to claims 18 or 19, characterized in that it constitutes apparatus for for treating prostate tissue by urethral or rectal route.

## Patentansprüche

1. Sonde (10') zur Abbildung und Therapie, aufweisend
- einen Sondenkörper (20') länglicher Form, die eine allgemeine Achse definiert (X-X),
- mindestens einen beweglich in der Sonde (20') befestigten Abbildungswandler (40') zum Aussenden von Bildwellen,
- mindestens einen beweglich in der Sonde befestigten Therapiewandler (30') zum Aussenden von Therapiewellen,
- wobei der Abbildungswandler (40') die Bildung einer Abbildung des mit dem Therapiewandler (30') zu behandelnden Zielbereichs (T.V.) erlaubt,
gekennzeichnet dadurch, daß der Abbildungswandler (40') beweglich in der Sonde (20') auf ersten Stützmitteln (26') befestigt ist, welche gewährleisten, daß die Verschiebung zwischen einer eingezogenen und einer vorgeschobenen Position des Abbildungswandlers im wesentlichen parallel zur allgemeine Achse der Sonde ist;
- daß der Therapiewandler (30') beweglich in der Sonde auf zweiten Stützmitteln (28') befestigt ist, welche gewährleisten, daß eine Rotation des Therapiewandlers um eine Rotationsachse (X1-X1), die im wesentlichen parallel zur allgemeine Achse (X-X) der Sonde, aber bezüglich des Abbildungswandlers versetzt ist;
- daß die zweiten Stützmittel gewährleisten, daß eine Rotation des Therapiewandlers (30') zwischen einer Behandlungsposition, in welcher der Abbildungswandler (40') sich in einer eingezogenen Lage befindet, und einer versenkten Position, Ruhelage genannt, in welcher der Abbildungswandler (40') in die vorgeschobenen Position verschoben werden kann und den von dem Therapiewandler (30') in seiner Bahandlungsposition eingenommenen Raum einnehmen kann ; und
- daß der Therapiewandler (30') in seiner Behandlungsposition Therapiewellen direkt in eine Richtung aussenden kann, die mit der Senderichtung der Abbildungswellen durch den Abbildungswandler in seiner vorgeschobenen Position im wesentlichen identisch ist.

2. Sonde zur Abbildung und Therapie (10') gemäß Anspruch 1, gekennzeichnet dadurch, daß sie Antriebsmittel (110') für den Abbildungswandler (40') und Antriebsmittel (100') für den Therapiewandler (30') aufweist.

3. Sonde zur Abbildung und Therapie (10') gemäß Anspruch 1 oder 2, gekennzeichnet dadurch, daß die zweiten Stützmittel eine Rotation des Therapiewandlers (30') außerhalb der Spannweite des Abbildungswandlers gewährleisten.

4. Sonde zur Abbildung und Therapie gemäß einem der Ansprüche 1 bis 3, gekennzeichnet dadurch, daß der Therapiewandler (30') und der Abbildungswandler (40') im Innern einer mit einer akustischen Kupplungsflüssigkeit wie etwa Wasser oder Öl gefüllten, flexiblen Membran (120') angeordnet sind.

5. Sonde zur Abbildung und Therapie gemäß einem der Ansprüche 1 bis 4, gekennzeichnet dadurch, daß der Therapiewandler (30') in einem mit den zweiten Stützmitteln (28') einstückigen Stützorgan (31') angebracht ist, dessen Querschnitt in einer zu der allgemeinen Achse (X-X) der Sonde senkrechten Ebene einen Umkreis definiert, der dem Umkreis des Querschnitts in derselben Ebene des Therapiewandlers (30') so nah wie möglich kommt.

6. Sonde zur Abbildung und Therapie gemäß Anspruch 5, gekennzeichnet dadurch, daß der Therapiewandler (30') eine äußere Form einer größtenteils im wesentlichen kreisförmigen Profilscheibe aufweist, aber mit einem kleineren Durchmesser in senkrechter Richtung bezüglich der allgemeinen Achse der Sonde.

7. Sonde zur Abbildung und Therapie gemäß einem der Ansprüche 1 bis 6, gekennzeichnet dadurch, daß der Abbildungswandler (40') dafür vorgesehen ist, Abbildungen in mehreren Ebenen (PR, PS), insbesondere in zwei senkrechten Ebenen, einer ersten Ebene (PR), genannt radial, senkrecht zur Hauptebene und einer zweite Ebene, genannt pfeilrecht, die allgemeine Achse enthaltend, der es insbesondere auch erlaubt, eine Abbildung des zu behandelnden Bereichs während des Behandlung durchzuführen.

8. Sonde zur Abbildung und Therapie gemäß einem der Ansprüche 1 bis 7, gekennzeichnet dadurch, daß der Therapiewandler (30') dafür vorgesehen ist, eine Fokussierung gemäß einer im wesentlichen senkrechten Richtung bezüglich der allgemeinen Achse durchzuführen.

9. Sonde zur Abbildung und Therapie gemäß einem der Ansprüche 1 bis 8, gekennzeichnet dadurch, daß der Therapiewandler (30') auf einem Stützorgan (31') angebracht ist, das eine im wesentlichen kreisförmige ode polygonale äußere Form aufweist, der äußeren Form des Therapiewandlers verwandt, welche selbst im wesentlichen kreisförmig oder polygonal ist, wobei das Stützorgan des Therapiewandlers in der Nähe eines Randes mit den zweiten Stützmitteln (28') einstückig ist, derart, daß die Verschiebung des Therapiewandlers (30') zwischen der versenkten Position und der Behandlungsposition erleichtert wird.

10. Sonde zur Abbildung und Therapie gemaß Anspruch 5, gekennzeichnet dadurch, daß der Therapiewandler (30') eine äußere Form aufweist, die eine Hauptebene definiert, welche in der Behandlungsposition die allgemeine Achse enthält.

11. Sonde zur Abbildung und Therapie gemäß einem der Ansprüche 1 bis 10, gekennzeichnet dadurch, daß diese manuelle oder motorisierte Drehantriebsmittel (100') des Therapiewandlers (30') um seine Achse, sowie manuelle oder motorisierte Verschiebungsmittel zur (110') des Abbildungswandlers (40') aufweist.

12. Sonde zur Abbildung und Therapie gemäß einem der Ansprüche 1 bis 11, gekennzeichnet dadurch, daß der Therapiewandler (30') ein Verhältnis von Durchmesser zu Brennweite aufweist, das zwischen 0,8 und 1,5 liegt, und besser um 1 liegt.

13. Sonde zur Abbildung und Therapie gemäß einem der Ansprüche 1 bis 12, gekennzeichnet dadurch, daß der Therapiewandler (30') gepulste Ultraschallwellen aussendet, mit einer Frequenz von 1 bis 4 MHz und einer kurzen Pulslänge zwischen 50 ms und 5 s, vorzugsweise zwischen 1 und 4 s, wobei die Intensität am Brennpunkt F zwischen 1 000 und 10 000 W/cm² liegt.

14. Sonde zur Abbildung und Therapie gemäß einem der Ansprüche 1 bis 13, gekennzeichnet dadurch, daß der Therapiewandler (60) eine mechanisch verformbare Linse (62) umfaßt, welche eine Änderung seiner Brennweite ermöglicht.

15. Sonde zur Abbildung und Therapie gemäß einem der Ansprüche 1 bis 14, gekennzeichnet dadurch, daß diese eine starre Wand umfaßt, die die Wandler zumindest teilweise umgibt.

16. Sonde zur Abbildung und Therapie gemäß Anspruch 15, gekennzeichnet dadurch, daß diese Wand ein Fenster aufweist.

17. Sonde zur Abbildung und Therapie gemäß einem der Ansprüche 1 bis 16, gekennzeichnet dadurch, daß diese eine flexible, örtlich verformbare Hülle aufweist.

18. Behandlungsgerät umfassend eine Therapie- und Abbindungssonde, derart wie sie in mindestens einem der vorstehenden Ansprüche angegeben sind.

19. Behandlungsgerät gemäß Anspruch 18, für die Behandlung eines gezielten Bereichs, insbesondere eines zu behandelnden Gewebebereichs und vor allem von bösartigen oder gutartigen Geweben, in der Regel in Form von gutartigen oder bösartigen Tumoren, im Körperinnern eines Säugetieres, insbesondere eines Menschen, umfassend die Mittel zur Einführung des Sonde in eine Körperhöhlung.

20. Behandlungsgerät gemäß einem der Ansprüche 18 oder 19, gekennzeichnet dadurch, daß diese ein Gerät zur Behandlung von Gewebe der Prostata auf urethalem oder rektalem Weg darstellt.
